# EUROPEAN PATENT SPECIFICATION

(11) **EP 1 200 129 B1**
(45) Date of publication and mention of the grant of the patent: **15.10.2008**
(21) Application number: 00945340.8
(22) Date of filing: 12.07.2000
(51) Int. Cl.: A61K 47/32

(54) **TASTE MASKING OF ORAL QUINOLONE LIQUID PREPARATIONS USING ION EXCHANGE RESINS**
MASKIEREN DES GESCHMACKS DER ORALEN FLÜSSIGEN CHINOLON-ZUBEREITUNGEN MIT IONENAUSTAUSCHERHARZEN
MASQUAGE DU GOUT DE PREPARATIONS LIQUIDES ORALES A BASE DE QUINOLONES AU MOYEN DE RESINES ECHANGEUSES D'IONS

(30) Priority: 14.07.1999 US 353549
(43) Date of publication of application: 02.05.2002
(73) Proprietor: Schering-Plough Ltd., 6004 Lucerne (CH)
(72) Inventor: GAO, Rong, Edison, NJ 08820 (US); SHAO, Zezhi, Jesse, Basking Ridge, NJ 07920 (US); FAN, Allan, Chor-Lun, Berkeley Heights, NJ 07922 (US); WITCHEY-LAKSHMANAN, Leonore, Catherine, Piscataway, NJ 08854 (US); STEWART, Daniel Charles, Cherry Hill, NJ 08034 (US)
(74) Representative: UEXKÜLL & STOLBERG
(86) International application number: PCT/US2000/018948
(87) International publication number: WO 2001/005431

(56) References cited:
- WO-A-93/05816
- US-A- 5 032 393
- US-A- 5 152 986
- US-A- 5 275 820

## Description

### BACKGROUND OF THE INVENTION

### 1. Field Of The Invention

The present invention relates to the formulation of oral liquid preparations of quinolones or derivatives thereof, selected from orbifloxacin, grepafloxacin, nalidixic acid, sparfloxacin and trovafloxacin mesylate, using ion exchange resins, selected from a cationic ion exchange resin made from porous copolymer of methacrylic acid crosslinked with divinylbenzene; and a sodium polystyrene sulfonate ion exchange resin, as the carrier. The formation of a quinolone-resin complex (resinate) eliminates the extreme bitterness of the quinolones to make the liquid oral dosage form palatable.

### 2. Description Of Related Art

Quinolone antibiotics are widely used in the treatment of common infections. The current quinolone products on the market, including orbifloxacin, and ciprofloxacin, are administered as tablets or capsules. Since quinolones have an extremely bitter taste, development of palatable liquid oral dosage forms has always been challenging. Liquid oral dosage forms are useful for patients having difficulty swallowing capsules or tablets. The Journal of Pharm. Sciences, vol 60, No 10, pp 1523-1527 (Oct 1971) discloses polycarboxylic acid ion exchange resins as adsorbates for masking the bad taste of ephedrine, dextromethorphan, pseudoephedrine, and methapyrilene; EPO 225615, published June 16, 1987, discloses liquid pharmaceutical compositions containing dextrometorphan, ion exchange resin (preferably a cationic resin) and acceptable pharmaceutical carriers, sweetners and formulation aids. Taste is not an issue. US 4,808,411 issued February 28, 1989 discloses antibiotic polymer compositions containing acrylic acid polymers and erythromycin. Said compositions can be prepared as liquids and are effective in masking the taste of the erythromycin antibiotic; US 5,152,986, issued October 6, 1992, discloses pharmaceutical compositions containing quinolone carboxylic acid derivatives (such as ciprofloxacin) and ion exchange resins (preferably cationic) which mask the bad taste of the quinolone in animal feeds. Said compositions are in solid form and paste form; EPO 622083, published November 11, 1994, discloses a solid pharmaceutical preparation containing any number of therapeutic agents, such as β-lactam antibiotics, antihistamines, bronchodilators and antiinflammatories, and cationic or anionic ion exchange resins which decrease the unpleasant taste and odor of the therapeutic agent.

There is still a need in the art for oral liquid quinolone preparations with acceptable taste. Applicants have satisfied this need in the art by preparing liquid quinolone preparations with acceptable taste.

### DEFINITIONS AND USAGES OF TERMS

The term "pharmaceutical composition", as used herein, means a combination comprised of a safe and effective amount of the quinolone compound active ingredient, or mixtures thereof, and pharmaceutically-acceptable excipients.

The term "pharmaceutically acceptable excipients", as used herein, means any physiologically inert, pharmacologically inactive material known to one skilled in the art, which is compatible with the physical and chemical characteristics of the particular quinolone compound active ingredient selected for use. Pharmaceutically-acceptable excipients include, but are not limited to, polymers, resins, plasticizers, fillers, binders, lubricants, glidants, disintegrants, solvents, co-solvents, buffer systems, surfactants, preservatives, sweetening agents, flavoring agents, pharmaceutical grade dyes or pigments, and viscosity agents.

The term "ion exchange resin", as used herein, means anionic or cationic ion exchange resins.

The term " oral dosage form", as used herein, means any pharmaceutical composition intended to be systemically administered to an individual by delivering said composition to the gastrointestinal tract of an individual, via the mouth of said individual. Oral dosage forms include, tablets, coated or non-coated; liquids, such as solutions and suspensions; or capsules, coated or non-coated.

All percentages are on a weight percent basis unless otherwise indicated.

### BRIEF SUMMARY OF THE INVENTION

The present invention relates to an aqueous pharmaceutical composition comprising:
(a) 0.1% to 10% by weight of a quinolone compound; wherein the quinolone compound is selected from orbifloxacin, grepafloxacin, nalidixic acid, sparfloxacin and trovafloxacin mesylate;
(b) 0.2% to 20% by weight of a cationic ion exchange resin made from porous copolymer of methacrylic acid crosslinked with divinylbenzene; and
(c) pharmaceutically acceptable exipients to equal 100%.

The present invention further relates to aqueous pharmaceutical composition consisting of
purified water 33,75% (w/v);
orbifloxacin 2% (w/v);
lactic acid to pH 4.5;
sodium polystyrene sulfonate ion exchange resin (USP) 12% (w/v);
malt extract 65% (w/v);
propylene glycol 2.5% (w/v);
sorbic acid 0.1% (w/v);
purified water to equal 100%.

### DETAILED DESCRIPTION OF THE INVENTION

The present invention relates to an aqueous pharmaceutical composition comprising:
(a) 0.1% to 10% by weight of a quinolone compound; wherein the quinolone compound is selected from orbifloxacin, grepafloxacin, nalidixic acid, sparfloxacin and trovafloxacin mesylate;
(b) 0.2% to 20% by weight of a cationic ion exchange resin made from porous copolymer of methacrylic acid crosslinked with divinylbenzene; and
(c) pharmaceutically acceptable exipients to equal 100%.

The present invention further relates to an aqueous pharmaceutical composition consisting of
purified water 33,75% (w/v);
orbifloxacin 2% (w/v);
lactic acid to pH 4.5;
sodium polystyrene sulfonate ion exchange resin (USP) 12% (w/v);
malt extract 65% (w/v);
propylene glycol 2.5% (w/v);
sorbic acid 0.1% (w/v);
purified water to equal 100%.

### Quinolones and Derivatives Thereof Useful in the Practice of the Present Invention

Quinolones and derivatives thereof useful in the practice of the present invention include orbifloxacin, grepafloxacin, nalidixic acid, ofloxacin,sparfloxacin, and trovafloxacin mesylate. The preferred quinolone is orbifloxacin available from Schering Plough, Kenilworth, NJ as ORBAX^{®}.

The quinolone compounds useful in the practice of the present invention comprise from 0.1 % to 10% by weight of the pharmaceutical compositions of the present invention. More preferably, the quinolone compounds useful in the practice of the present invention comprise from about 0.5% to 5 % by weight of the pharmaceutical compositions of the present invention

### Ion Exchange Resins Useful in the Practice of the Present Invention

Ion exchange resins useful in the practice of the present invention include cationic resins such as: AMBERLITE^{®} IRP-64 (a porous copolymers of methacrylic acid crosslinked with divinylbenzene), and AMBERLITE^{®}IRP-69 (Sodium polystyrene sulfonate USP). AMBERLITE^{®} IRP 64 is preferred resin. The AMBERLITE^{®} resins are available from the Rohm and Haas Company, Philadelphia, PA. The DOWEX^{®} resins, available from the Dow Chemical Company, Midland, MI are also useful in the practice of the present invention. Said DOWEX^{®} resins are strong cationic exchangers based upon polystyrenesulphonic acid with variable crosslinking (1-12% divinylbenzene) in a variety of particle sizes.

Further, said AMBERLITE^{®} IRP 69 (sodium polystyrenesulfonate) is available commercially as a sodium salt. However, it is within the scope of the present invention to convert the sodium salt to other salt forms including, but not limited to, K and Li.

The ion exchange resins useful in the practice of the present invention comprise from 0.2 % to 20% by weight of the pharmaceutical compositions of the present invention. More preferably, the ion exchange resins useful in the practice of the present invention comprise from about 0.5% to 15% by weight of the pharmaceutical compositions of the present invention

### Pharmaceutically Acceptable Excipients Useful in the Practice of the Present Invention

As stated hereinabove, pharmaceutically-acceptable excipients include, but are not limited to, resins, fillers, binders, lubricants, solvents, glidants, disintegrants, co-solvents, surfactants, preservatives, sweetener agents, flavoring agents, buffer systems, pharmaceutical-grade dyes or pigments, and viscosity agents.

The solvent is water.

Flavoring agents among those useful herein include those described in Remington's Pharmaceutical Sciences, 18th Edition, Mack Publishing Company, 1990, pp. 1288-1300, incorporated by reference herein. The pharmaceutical compositions suitable for use herein generally contain from 0-5% flavoring agents.
Preferred co-solvents include, but are not limited to, ethanol, glycerin, propylene glycol, polyethylene glycols. The pharmaceutical compositions of the present invention include from 0.01 % to 30% co-solvents.

Preferred buffer systems include, but are not limited to, NaOH, acetic, boric, carbonic, phosphoric, succinic, malaic, tartaric, citric, benzoic, lactic, glyceric, gluconic, glutaric and glutamic acids and their sodium, potassium and ammonium salts. The pharmaceutical composition of the present invention generally contain from 0.1 % to 20% buffer systems.

Preferred surfactants include, but are not limited to, polyoxyethylene sorbitan fatty acid esters, polyoxyethylene monoalkyl ethers, sucrose monoesters and lanolin esters and ethers, alkyl sulfate salts, sodim, potassium, and ammonium salts of fatty acids.

Preferred preservatives include, but are not limited to, phenol, alkyl esters of parahydroxybenzoic acid, sorbic acid, and methylparaben, o-phenylphenol benzoic acid and the salts thereof, chlorobutanol, benzyl alcohol, thimerosal, phenylmercuric acetate and nitrate, nitromersol, benzalkonium chloride, cetylpyridinium chloride, methyl paraben, and propyl paraben. Particularly preferred is sorbic acid. The compositions of the present invention generally include from 0.01 % to 5% preservatives.

Preferred sweeteners include, but are not limited to, sucrose, glucose, saccharin, sorbitol, malt extract syrup, mannitol, and aspartame. Particularly preferred is malt extract syrup. Sweeteners such as sucrose, glucose, saccharin and sorbitol are generally used at levels of 0.1 % to 10%. Sweeteners such as malt extract syrup are generally used at levels of 10% to 75%.

Preferred viscosity agents include, but are not limited to, methylcellulose, sodium carboxymethylcellulose, hydroxypropyl-methylcellulose, hydroxypropylcellulose, sodium alginate, carbomer, povidone, acacia, guar gum, xanthan gum and tragacanth. Particularly preferred are methylcellulose, carbomer, xanthan gum, guar gum, povidone, sodium carboxymethylcellulose, and magnesium aluminum silicate. Compositions of the present invention include 0.1 % to 5% viscosity agents.

The compositions of the present invention may optionally contain lactose, mannitol, sorbitol, tribasic calcium phosphate, dibasic calcium phosphate, compressible sugar, starch, calcium sulfate, dextro and microcrystalline cellulose, magnesium stearate, stearic acid, talc, colloidal silicon dioxide, starch, sodium starch glycolate, crospovidone, croscarmelose sodium, and microcrystalline cellulose, acacia, tragacanth, hydroxypropylcellulose, pregelantinized starch, gelatin, povidone, ethylcellulose, hydroxypropylcellulose, hydroxypropylmethylcellulose, and methylcellulose.

### Preparing the Compositions of the Present Invention

The compositions of the present invention are prepared according to methods known to those skilled in the art. Basically, the preparation procedure involves dissolving the quinolone in an aqueous media followed by the addition of the ion exchange resin to form a drug/resin complex. The complex can be suspended directly into suitable vehicles with flavor agents such as, but not limited to, a syrup base (malt extract) with the aid of an anticaking agent such as, but not limited to, colloidal silicone dioxide and a preservative, such as, but not limited to, sorbic acid.

The drug/resin complex can also be isolated and dried for later usage. This would be advantageous when reconstitution in the pharmacy is desired or very bitter drugs are being employed. Specifically, the quinolone and ion exchange resin complex can be blended with, for example, lactose, magnesium stearate, silicon dioxide, talc, microcrystalline cellulose or gelatin, to prepare a powder that can be shipped to the pharmacy and reconstituted into a palatable oral liquid dosage form by the pharmacist. For very bitter drugs, the drug/resin complex can be isolated, for example, by rinsing with deionized water, from uncomplexed (or free) drug. The isolated and dried powder will contain substantially only drug/resin complex. This isolated, purer drug/resin complex (substantially devoid of free drug) can be formulated into an oral liquid preparation that contains little to no amount of the bitter free drug.

The following nonlimiting Examples 1 & 2 illustrate the compositions of the present invention. Said Examples are prepared on a weight to volume (w/v) basis.

### Example 1

Purified water, USP pure - 33.75%
Orbifloxacin - 2%
Lactic Acid, USP to pH 4.5
Sodium Polystyrene Sulfonate ion exchange resin (USP) - 12%
Malt Extract - 65%
Propylene Glycol - 2.5%
Sorbic Acid - 0.1%
Purified water, USP Pure to equal 100%

The general procedure for preparing the composition described in Example 1 is as follows:
1) Charge orbifloxacin into water and mix well.
2) Add Lactic acid and adjust pH to 4.5
3) Charge sodium polystyrene sulfonate resin and mix well to form a slurry .
4) Charge malt extract syrup to slurry and mix well.
5) Dissolve sorbic acid in propylene glycol and charge it into slurry formed in steps 3 and 4.
6) Add water to equal 100%, weight to volume.

### Example 2

Purified Water, USP - 45%
Orbifloxacin - 3%
Lactic Acid to pH, 4.5
AMBERLITE IRP-64 - 15%
50% w/w NaOH to pH 5.5
Sorbic Acid - 0.1%
Propylene Glycol - 10%
Colloidal Silicon Dioxide - 1.5%
Malt Extract to equal 100%

The general procedure for preparing the composition described in Example 2 is as follows:
1) Charge orbifloxacin into water and mix well.
2) Add Lactic acid to adjust pH to 4.5
3) Charge Amberlite^{®} IRP-64 and mix well to form a slurry.
4) Adjust pH to 5.5 by adding 50% w/w NaOH.
5) Dissolve sorbic acid in propylene glycol and add it to the pH adjusted slurry.
6) Add colloidal silicon dioxide and mix well.
7) Add malt extract syrup to equal 100%, weight to volume.

## Claims

1. An aqueous pharmaceutical composition comprising:
(a) 0.1% to 10% by weight of a quinolone compound; wherein the quinolone compound is selected from orbifloxacin, grepafloxacin, nalidixic acid, sparfloxacin and trovafloxacin mesylate;
(b) 0.2% to 20% by weight of a cationic ion exchange resin made from porous copolymer of methacrylic acid crosslinked with divinylbenzene; and
(c) pharmaceutically acceptable exipients to equal 100%.

2. The composition according to claim 1 wherein the quinolone is orbifloxacin.

3. The composition according to claim 2 wherein (a) the orbifloxacin is 0.5% to 5% by weight; and (b) the cationic ion exchange resin is 0.5% to 15% by weight.

4. The aqueous pharmaceutical composition according to claim 1 consisting of
purified water 45% (w/v);
orbifloxacin 3%;
lactic acid to pH 4.5;
AMBERLITE IRP-64 15% (w/v);
50% (w/w) NaOH to pH 5.5;
sorbic acid 0.1% (w/v);
propylene glycol 10% (w/v);
colloidal silicon dioxide 1.5% (w/v);
malt extract to equal 100%.

5. An aqueous pharmaceutical composition consisting of
purified water 33,75% (w/v);
orbifloxacin 2% (w/v);
lactic acid to pH 4.5;
sodium polystyrene sulfonate ion exchange resin (USP) 12% (w/v);
malt extract 65% (w/v);
propylene glycol 2.5% (w/v);
sorbic acid 0.1% (w/v);
purified water to equal 100%.

## Patentansprüche

1. Wässrige pharmazeutische Zusammensetzung, umfassend:
a. 0,1 bis 10 Gew.-% einer Chinolonverbindung, wobei die Chinolonverbindung aus der Gruppe bestehend aus Orbifloxacin, Grepafloxacin, Nalidixinsäure, Sparfloxacin und Trovafloxacin-Mesylat besteht;
b. 0,2 Gew.-% bis 20 Gew.-% eines Kationen-Austauschharzes aus einem porösen, mit Divinylbenzol vernetztem Copolymer der Methacrylsäure;
c. pharmazeutisch verträgliche Hilfsstoffe bis auf 100 %.

2. Wässrige pharmazeutische Zusammensetzung gemäß Anspruch 1, in der die Chinolonverbindung Orbifloxazin ist.

3. Wässrige pharmazeutische Zusammensetzung gemäß Anspruch 2, in der (a) das Orbifloxazin in einer Konzentration von 0,5 bis 5 Gew.-% vorliegt; und (b) das Kationen-Austauschharz in einer Konzentration von 0,5 bis 15 Gew.-% vorliegt.

4. Wässrige pharmazeutische Zusammensetzung gemäß Anspruch 1, bestehend aus
45 Gew.% gereinigtem Wasser;
Orbifloxacin 3%;
Milchsäure bis zu pH 4,5;
Amberlite IRP-64 15 Gew.-%;
Sorbinsäure 0,1 Gew.-%;
Propylenglykol 10 Gew.-%;
Malzextrakt bis zu 100%.

5. Wässrige pharmazeutische Zusammensetzung bestehend aus
33,75 Gew.% gereinigtem Wasser;
Orbifloxacin 2 Gew.-%;
Milchsäure bis zu pH 4,5;
Natriumpolystyrolsulfonat Ionenaustauschharz (USP), 12 Gew.-%;
Malzextrakt 65 Gew.-%;
Propylenglykol 2,5 Gew.-%;
Sorbinsäure 0,1 Gew.-%;
gereinigtes Wasser bis zu 100%.

## Revendications

1. Composition pharmaceutique aqueuse comprenant :
a) de 0,1 à 10 % en poids d'un composé de type quinolone, lequel composé de type quinolone est choisi parmi l'orbifloxacine, la grépafloxacine, l'acide nalidixique, la sparfloxacine et le mésylate de trovafloxacine ;
b) de 0,2 à 20 % en poids d'une résine échangeuse de cations, faite d'un copolymère poreux d'acide méthacrylique, réticulé à l'aide de divinyl-benzène ;
c) et des excipients pharmacologiquement admissibles, pour complément à 100 %.

2. Composition conforme à la revendication 1, dans laquelle la quinolone est de l'orbifloxacine.

3. Composition conforme à la revendication 2, dans laquelle il y a
a) de 0,5 à 5 % en poids d'orbifloxacine,
b) et de 0,5 à 15 % en poids de résine échangeuse de cations.

4. Composition pharmaceutique aqueuse conforme à la revendication 1, constituée des composants suivants :
eau purifiée : 45 % p/v ;
orbifloxacine : 3 % ;
acide lactique : jusqu'à pH 4,5 ;
résine Amberlite IRP-64 : 15 % p/v ;
solution à 50 % p/p de NaOH : jusqu'à pH 5,5 ;
acide sorbique : 0,1 % p/v ;
propylèneglycol : 10 % p/v ;
silice colloïdale : 1,5 % p/v ;
extrait de malt : pour compléter à 100 %.

5. Composition pharmaceutique aqueuse constituée des composants suivants :
eau purifiée : 33,75 % p/v ;
orbifloxacine : 2 % p/v ;
acide lactique : jusqu'à pH 4,5 ;
résine échangeuse d'ions à base de polystyrène-sulfonate de sodium (USP) : 12 % p/v;
extrait de malt : 65 % p/v ;
propylèneglycol : 2,5 % p/v ;
acide sorbique : 0,1 % p/v ;
eau purifiée : pour compléter à 100 %.
